(19) Europäisches Patentamt / European Patent Office / Office européen des brevets

(11) **EP 4 188 300 B1**

(12) **EUROPEAN PATENT SPECIFICATION**

(45) Date of publication and mention
of the grant of the patent:
**10.12.2025 Bulletin 2025/50**

(21) Application number: **20946961.8**

(22) Date of filing: **31.07.2020**

(51) International Patent Classification (IPC):
***A61F 13/49*** *(2006.01)*

(52) Cooperative Patent Classification (CPC):
**A61F 13/4902; A61F 13/49011**

(86) International application number:
**PCT/CN2020/106037**

(87) International publication number:
**WO 2022/021265 (03.02.2022 Gazette 2022/05)**

(54) **WEARABLE ARTICLE COMPRISING AN ELASTIC BELT LAMINATE HAVING HYDROPHILIC INNER AND OUTER WEB**

TRAGBARER ARTIKEL MIT EINEM ELASTISCHEN GÜRTELLAMINAT MIT HYDROPHILEN INNEREN UND ÄUSSEREN BAHNEN

ARTICLE PORTABLE COMPRENANT UN STRATIFIÉ DE CEINTURE ÉLASTIQUE AYANT DES BANDES INTERNES ET EXTERNES HYDROPHILES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**07.06.2023 Bulletin 2023/23**

(73) Proprietor: **The Procter & Gamble Company Cincinnati, OH 45202 (US)**

(72) Inventors:
• **ROSATI, Rodrigo**
  **65824 Schwalbach am Taunus (DE)**

• **MORIMOTO, Koichi**
  **Beijing 101312 (CN)**
• **LIU, Hui**
  **Beijing 101312 (CN)**

(74) Representative: **P&G Patent Germany Procter & Gamble Service GmbH Sulzbacher Straße 40 65824 Schwalbach am Taunus (DE)**

(56) References cited:
**WO-A1-2019/169989    WO-A1-2019/169989
CN-A- 102 316 838    CN-A- 110 072 499
CN-A- 110 087 600    US-A1- 2019 274 895**

**Description**

FIELD OF THE INVENTION

The present invention relates to an elastic laminate which forms an elastic belt of a wearable article, wherein the elastic belt exhibits improved sweat management properties.

BACKGROUND OF THE INVENTION

Substrate materials such as nonwoven fabrics and laminates thereof, are commonly used for wearable articles such as absorbent articles. For example, absorbent articles typically use nonwoven substrate materials for both the skin facing side as well as the garment facing side of the articles, to control the movement of liquids and to provide a comfortable, conforming fit when the article is worn by a wearer. By comfortableness, what may be desired is a cloth-like substrate which is capable of effectively absorbing sweat and excess moisture from the skin and releasing them outside the article. Such is particularly desired, but not limited to, for absorbent articles by caregivers of young children, wherein skin health is closely associated with the absence of heat rashes and diaper rashes, but also with absorbent articles, such as pants, for incontinent adults, which are often older and also may have delicate skin. Heat rashes in the waist area may be associated with wetness or dampness in the waist area inside an absorbent article. It is a common practice for caregivers to check the degree of wetness or dampness by touching the waist area inside the absorbent article worn by a young child.

Elastic laminates having sweat management properties have been proposed, such as those described in Japanese Patent Application publications 2017-12319A and 2017-113186A. There is a need to provide elastic laminates with further improved sweat management properties, while being economic to make.

WO2019169989 discloses a wearable article comprising an elastic laminate. The elastic laminate comprises an inner first nonwoven layer and an outer second nonwoven layer. The outer layer has a higher hydrophilicity than the inner layer. The laminate has a total caliper when subjected to the NMR MOUSE method as described.

US20190274895 relates to a wearable article comprising an elastic laminate. The elastic laminate comprises an inner first nonwoven layer and an outer second nonwoven layer. The outer layer has a higher hydrophilicity than the inner layer. The laminate has a total caliper when subjected to the NMR MOUSE method as described. The total caliper has a first sub-caliper corresponding to 50% of the total caliper, and a second sub-caliper corresponding to the remaining 50% of the total caliper. The elastic laminate has a Liquid Ratio of the first sub-caliper to the second sub-caliper of at least 1.1 after 1 minute according to the NMR MOUSE method.

CN 102316838 is concerned with a disposable diaper equipped with a central absorbent body spanning from a back-side section to an abdomen-side section, and with waist flaps having a hydrophilic sheet disposed therein. The hydrophilic sheet has an inner hydrophilic sheet and an outer hydrophilic sheet disposed on the side of a surface not in contact with the skin.

Elastic laminates used, e.g. for belts of absorbent pants are typically made of two nonwoven webs, which are joined to each other in a face to face relationship, with elastic strands sandwiched in between. In such laminates, a first nonwoven web (the inner nonwoven web) is facing the skin of the wearer and is in direct contact with the skin over a relatively large area of its surface. A second nonwoven web (the outer nonwoven web) is facing outwardly, away from the wearer, so it will commonly be in contact with the garment of the wearer. Very often, the outer nonwoven web comprises an extended portion which is folded over the inner nonwoven web at the edge of the elastic laminate that forms the waist edge of the absorbent pant. The extended, folded over portion of the outer web extends from the waist edge (which is formed by the fold) toward the crotch area along the longitudinal dimension of the wearable article. Thereby, a more underwear-like, finished appearance of the pant is provided. Consequently, adjacent to the waist edge, the elastic laminate may comprise three nonwoven webs, namely the inner nonwoven web which is sandwiched between the outer nonwoven web and the extended, folded over portion of the outer nonwoven web. It may be desirable that the extended, folded over portion of the outer web extends toward the crotch area to a relatively large degree. In such circumstances, the sweat management will be considerably impacted by an appropriate configuration of the area covered by the three nonwoven webs. Hence, there is a need for an elastic belt which pays special attention to the areas having three nonwoven webs.

The first and second nonwoven webs are typically both hydrophobic which has been found to lead to a relatively unsatisfactory performance in sweat management, i.e. in transporting sweat from the skin of the wearer through the laminate to the outside.

To address this drawback, it has been suggested to use a hydrophobic inner nonwoven web and a hydrophilic outer nonwoven web. However, in an elastic laminate of a pant belt having an extended, folded over portion of the outer nonwoven web at least a portion of the folded over portion will typically be in direct contact with a wearer's skin. In such configurations, transport of sweat from a wearer's skin tends to be reduced by the hydrophobic inner nonwoven web which is sandwiched between the hydrophilic folded over portion of the outer nonwoven web and the hydrophilic outer nonwoven web.

Hence, there is a need for an elastic belt which pays special attention to the areas having three nonwoven webs with regard to sweat management.

SUMMARY OF THE INVENTION

The invention relates to a wearable article having a longitudinal direction extending from a front waist edge to a back waist edge, and a transverse direction extending perpendicular to the longitudinal direction.

The wearable article comprises an elastic laminate which forms an elastic belt with a front belt and a back belt. The front and back belt are discontinuous in the longitudinal direction of the article. Left and right transverse edges of the front belt and the back belt are joined by side seams to form two leg opening. The front and back waist edge jointly form a continuous waist opening. The elastic laminate comprises an inner web and an outer web being in face to face relationship with each other. The inner web and outer web are comprised by the complete surface area of the elastic laminate. The inner web and the outer web are each formed of a hydrophilic nonwoven web comprising at least 90 weight-% synthetic fibers based on the total weight of the respective inner web and outer web. As defined below, "hydrophilic" means that the inner and outer web each have a contact angle of less than 90°, as measured according to the test method set out below. Each of the inner and outer web may also have a contact angle of less than 70°, or less than 60°, as measured according to the test method set out below.

At the front and back waist edge, the elastic laminate comprises a fold over region wherein the outer web is extended beyond the elastic laminate, and the extended portion of the outer web is folded over the inner web, such that a portion of the elastic belt comprises the inner web sandwiched between the outer web and the extended portion of the outer web. The extended folded over portion has a longitudinal extension parallel with the longitudinal direction of the wearable article. The longitudinal extension of the extended folded over portion has a dimension on the longitudinal direction of the wearable article which is from 20% to 70% of the total length of the side seams.

The total length of the side seams can be determined from the waist opening to the leg opening along a straight line along the side seams.

BRIEF DESCRIPTION OF THE DRAWINGS

    Figures 1A-1B are a schematic plan views of one embodiment of a wearable article of the present invention with the seams un-joined and in a flat uncontracted condition showing the garment facing surface

    Figures 2A-2C are schematic cross section views of embodiments of wearable articles of the present invention

DEFINITIONS

As used herein, the following terms shall have the meaning specified thereafter:

"Wearable article" refers to articles of wear which may be in the form of pants, taped diapers, incontinent briefs, feminine hygiene garments, wound dressings, hospital garments, and the like. Preferably, the wearable article of the present invention is a pant. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.

As used herein, " taped diaper" and "pant" refers to an absorbent article generally worn by babies, infants and incontinent persons about the lower torso to encircle the waist and legs of the wearer and that is specifically adapted to receive and contain urinary and fecal waste. In a pant, as used herein, the longitudinal edges of the first and second waist region are attached to each other to a pre-formed waist opening and leg openings. A pant is generally placed in position on the wearer by inserting the wearer's legs into the leg openings and sliding the pant absorbent article into position about the wearer's lower torso. A pant may be pre-formed by any suitable technique including, but not limited to, joining together portions of the absorbent article using refastenable and/or non-refastenable bonds (i.e. with permanent side seams not intended to be torn upon prior to removal of the pant from the wearer for disposal). In a diaper, the waist opening and leg openings are only formed when the diaper is applied onto a wearer by (releasable) attaching the longitudinal edges of the first and second waist region to each other on both sides by a suitable fastening system.

"Taped diaper" refers to disposable absorbent articles which are applied on a wearer by tapes.

As used herein, "disposable" is used in its ordinary sense to mean an article that is disposed or discarded after a limited number of usage over varying lengths of time, for example, less than 20 usages, less than 10 usages, less than 5 usages, or less than 2 usages. If the disposable absorbent article is a taped diaper, a pant, sanitary napkin, sanitary pad or wet wipe for personal hygiene use, the disposable absorbent article is most often intended to be disposed after single use.

The absorbent articles described herein are disposable.

"Longitudinal" refers to a direction running substantially perpendicular from a waist edge to an opposing waist edge of the article and generally parallel to the maximum linear dimension of the article. "Transverse" refers to a direction perpendicular to the longitudinal direction.

"Inner" and "outer" refer respectively to the relative location of an element or a surface of an element or group of elements. "Inner" implies the element or surface is nearer to the body of the wearer during wear than some other element or surface. "Outer" implies the element or surface is more remote from the skin of the wearer during wear than some other element or surface (i.e., element or surface is more proximate to the wearer's garments that may be worn over the present article).

"Body-facing" (also referred to as "skin-facing" herein) and "garment-facing" refer respectively to the relative location of an element or a surface of an element or group of elements. "Body-facing" implies the element or surface is nearer to the wearer during wear than another element of the same component. An example is the inner layer of the elastic laminate of the present invention wherein the inner layer (being an element of the elastic laminate) is nearer to the body of the wearer than the outer layer (being another element of the elastic laminate). "Garment-facing" implies the element or surface is more remote from the wearer during wear than another element of the same component. The garment-facing surface may face another (i.e. other than the wearable article) garment of the wearer, other items, such as the bedding, or the atmosphere.

"Proximal" refers to a portion being closer relative to the longitudinal center of the article, while "distal" refers to a portion being farther from the longitudinal center of the article.

"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Hydrophilic" describes surfaces of substrates which are wettable by aqueous fluids (e.g., aqueous body fluids) deposited on these substrates. Hydrophilicity and wettability are typically defined in terms of contact angle and the strike-through time of the fluids, for example through a nonwoven fabric. This is discussed in detail in the American Chemical Society publication entitled "Contact Angle, Wettability and Adhesion", edited by Robert F. Gould (Copyright 1964). A surface of a substrate is said to be wetted by a fluid (i.e., hydrophilic) when either the contact angle between the fluid and the surface is less than 90°, or when the fluid tends to spread spontaneously across the surface of the substrate, both conditions are normally co-existing. Conversely, a substrate is considered to be "hydrophobic" if the contact angle is equal or greater than 90° and the fluid does not spread spontaneously across the surface of the fiber. The contact angle test method used for the present invention is set out herein below.

"Extendibility" and "extensible" mean that the width or length of the component in a relaxed state can be extended or increased.

"Elasticated" and "elasticized" mean that a component comprises at least a portion made of elastic material.

"Elongation rate" means the state of elongation of a material from its relaxed, original length, namely an elongation rate of 10% means an elongation resulting in 110% of its relaxed, original length.

"Elongatable material", "extensible material", or "stretchable material" are used interchangeably and refer to a material that, upon application of a biasing force, can stretch to an elongation rate of at least 10% (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastic." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "non-elastic". For example, an elongatable material that has an initial length of 100mm can extend at least to 150mm, and upon removal of the force retracts to a length of at least 145mm (i.e., exhibiting a 10% recovery).

As used herein, the term "nonwoven web" refers to a material which is a manufactured web/layer of directionally or randomly oriented fibers or filaments. The fibers may be of natural or man-made origin. Natural fibers may be selected from the group consisting of wheat straw fibers, rice straw fibers, flax fibers, bamboo fibers, cotton fibers, jute fibers, hemp fibers, sisal fibers, bagasse fibers, Hesper aloe fibers, miscanthus, marine or fresh water algae/seaweeds, silk fibers, wool fibers, and combinations thereof. Another group of fibers may also be regenerated cellulose fibers, such as viscose, Lyocell (Tencel®), rayon, modal, cellulose acetate fibers, acrylic fibers, cuprammonium rayon, regenerated protein fibers etc. Preferably, the natural fibers or modified natural fibers are selected from the group consisting of cotton fibers, bamboo fibers, viscose fibers or mixtures thereof. Preferably, the natural fibers are cotton fibers. Synthetic fibers may be selected from the group consisting of polyolefins (such as polyethylene, polypropylene or combinations and mixtures thereof), polyethylene terephthalate (PET), co PET, polylactic acid (PLA), polybutylene succinate (PBS), polyhydroxy alkanoid (PHA), nylon (or polyamide), or mixtures or combinations thereof. An alternative option is to use superabsorbent fibers, for example SAF™ which is a cross-linked terpolymer based on acrylic acid, which is partially neutralised to its sodium salt, commercially available from Technical Absorbents.

The fibers in a nonwoven web are consolidated by friction, and/or cohesion and/or adhesion, and/or by heat bonding, pressure bonding, heat and pressure bonding, and/or ultrasonic bond excluding paper and products which are woven, knitted, tufted, stitch-bonded. The fibers may be staple fibers (e.g. in carded nonwoven webs) or continuous fibers (e.g. in spunbonded or meltblown nonwoven webs).

Nonwoven webs can be formed by many processes such as meltblowing, spunlaying, solvent spinning, electrospinning, and carding, and the fibers can be consolidated, e.g. by hydroentanglement (in spunlaced nonwoven webs), air-through bonding (using hot air that is blown through the fiber layer in the thickness direction), needle-punching, one or more patterns of bonds and bond impressions created through localized compression and/or application of heat or ultrasonic energy, or a combination thereof. The fibers may, alternatively or in addition, be consolidated by use of a binder. The binder may be provided in the form of binder fibers (which are subsequently molten) or may be provided in liquid, such as a styrene butadiene binder. A liquid binder is provided to the fibers (e.g. by spraying, printing or foam application) and is subsequently cured to solidify.

The basis weight of nonwoven webs is usually expressed in grams per square meter (g/m$^2$ or gsm).

In a spunlace nonwoven web the fibers have been carded as precursor web and then subjected to hydroentanglement to intermingle and intertwine the fibers with each other. Cohesion and the interlacing of the fibers with one another may be obtained by means of a plurality of jets of water under pressure passing through a moving fleece or cloth and, like needles, causing the fibers to intermingle with one another (hereinafter also referred to as "hydraulic interlacing"). Thus, consolidation of a spunlace nonwoven web is essentially a result of hydraulic interlacing. "Spunlace nonwoven web", as used herein, also relates to a nonwoven formed of two or more precursor webs, which are combined with each other by hydraulic interlacing.

The two or more webs, prior to being combined into one nonwoven by hydraulic interlacing, may have underdone bonding processes, such as heat and/or pressure bonding by using e.g. a patterned calendar roll and an anvil roll to impart a bonding pattern. However, the two or more webs are combined with each other solely by hydraulic interlacing. Alternatively, the spunlace nonwoven web is a single web, i.e. it is not formed of two or more precursor webs. Still in another alternative, the spunlace nonwoven web of the present invention may be formed of one precursor web onto which staple fibers are laid down. The staple fibers may not have been consolidated into a self-sustaining precursor web, but the fibers are loosely laid onto the precursor web. The relatively loose staple fibers are then integrated and intertwined with each other and with the fibers of the underlying precursor web by (only) hydraulic interlacing. Spunlace nonwoven layers/webs can be made of staple fibers or continuous fibers (continuous fibers are also often referred to as filaments).

Through-air bonding (interchangeably used with the term "air-through bonding") means a process of bonding staple fibers or continuous fibers by forcing air through the nonwoven web, wherein the air is sufficiently hot to melt (or at least partly melt, or melt to a state where the fiber surface becomes sufficiently tacky) the polymer of a fiber or, if the fibers are multicomponent fibers, wherein the air is sufficiently hot to melt (or at least partly melt, or melt to a state where the fiber surface becomes sufficiently tacky) one of the polymers of which the fibers of the nonwoven web are made. The melting and re-solidification of the polymer provide the bonding between different fibers.

"Comprise," "comprising," and "comprises" are open ended terms, each specifies the presence of the feature that follows, e.g. a component, but does not preclude the presence of other features, e.g. elements, steps, components known in the art or disclosed herein. These terms based on the verb "comprise" encompasses the narrower terms "consisting essential of" which excludes any element, step or ingredient not mentioned which materially affect the way the feature performs its function, and the term "consisting of" which excludes any element, step, or ingredient not specified.

DETAILED DESCRIPTION OF THE INVENTION

Elastic laminate of the wearable article

The elastic laminate comprised by the wearable article of the present invention comprises an inner web and an outer web being in a face to face relationship. The inner web and outer web are comprised by the complete surface area of the elastic laminate. The elastic laminate forms an elastic belt of the wearable article.

The inner web and the outer web are formed of a hydrophilic nonwoven web comprising at least 90 weight-% synthetic fibers based on the total weight of inner and outer web in combination. The inner web and the outer web may each be formed of a hydrophilic nonwoven web comprising at least 90 weight-% synthetic fibers based on the total weight of inner and outer web, respectively. As defined above, "hydrophilic" means that the inner and outer web each have a contact angle of less than 90°, as measured according to the test method set out below. Each of the inner and outer web may also have a contact angle of less than 70°, or less than 60°, as measured according to the test method set out below.

The inner web and/or the outer web may be formed of 95 weight-% or of 100 weight-% of synthetic fibers. The fibers of the inner way may be the same as or different from the fibers of the outer web. E.g. the fibers of the inner web may differ from the fibers of the outer web by using different thermoplastic material, different types of fibers (such as monocomponent fibers, bicomponent fibers), different fiber shapes and/or different diameter of the fibers, different hydrophilic finishes (e.g. spraying or otherwise treating the fibers with a hydrophilic surfactant). Alternatively or in addition, the inner web may use a different combination or mixture of fibers than the outer web.

The synthetic fibers may be thermoplastic fibers. The synthetic fibers may be selected from the group consisting of polyethylene, polypropylene, polyester, polylactic acid (PLA), and mixtures and combinations (such as co-polymers of polyethylene and polypropylene) thereof.

The inner web may comprise up to 10 weight-%, or up to 5 weight-%, or up to 2 weight-% of natural fibers, modified natural fibers and/or superabsorbent fibers based on the total basis weight of the inner web (if mixtures of natural fibers, modified natural fibers and superabsorbent fibers -or only two of them- are comprised, the overall amount of these types of fibers together may be up to 10 weight-%, or up to 5 weight-%, or up to 2 weight-% based on the total basis weight of the inner web). The natural fibers or modified natural fibers may be hydrophilic fiber. They may be selected from the group consisting of cotton, bamboo, viscose, cellulose, silk, or mixtures or combinations thereof. Preferred modified natural hydrophilic fibers are regenerated cellulose fibers. E.g. viscose is a modified natural hydrophilic fiber in that it is made of regenerated cellulose fibers such as cellulose fibers from wood or bamboo or cotton.

Likewise, the outer web may comprise up to 10 weight-%, or up to 5 weight-%, or up to 2 weight-% of natural fibers, modified natural fibers and/or superabsorbent fibers based on the total basis weight of the outer web (if mixtures of natural fibers, modified natural fibers and superabsorbent fibers -or only two of them- are comprised, the overall amount of these types of fibers together may be up to 10 weight-%, or up to 5 weight-%, or up to 2 weight-% based on the total basis weight of the outer web). The natural fibers or modified natural fibers may be hydrophilic fiber. They may be selected from the group consisting of cotton, bamboo, viscose, cellulose, silk, or mixtures or combinations thereof. Preferred modified natural hydrophilic fibers are regenerated cellulose fibers. E.g. viscose is a modified natural hydrophilic fiber in that it is made of regenerated cellulose fibers such as cellulose fibers from wood or bamboo or cotton.

The use of smaller amounts of natural fibers (e.g. up to 10 weight-%) can contribute to improved sustainability of the article and is also considered healthier and more comfortable for the skin vs. synthetic fibers. However, while natural fibers readily absorb the fluid, such as a wearer's sweat, they do not easily transfer the liquid to other layers. Hence, nonwoven webs using higher amounts of natural fibers may potentially feel wet both on the skin of the wearer and on the surface facing the wearer's clothes.

In addition the presence of natural fibers, such as cotton or viscose, provides additional capacity for temporary storage of sweat until it is transported away via evaporation: in fact natural fibers, such as cotton and viscose, are known to be able to absorb moisture within the fiber itself at significantly higher levels than traditional synthetic fibers such as polypropylene or polyester.

At least a portion of the inner web (namely the portion forming the innermost surface of the wearable article) is in direct contact with the skin of the wearer when the article is in use. The outer web may form a part of an outermost surface of the wearable article, i.e. the surface which is facing towards the garment of the wearer in use. As the outer web comprises an extended portion which is folded over the inner web (as described in more detail below), this extended portion may contribute to the innermost surface of the wearable article and may be in direct contact with the skin of the wearer when the article is in use.

The outer nonwoven web may be a carded, air-through bonded layer formed of staple fibers. Alternatively, the outer nonwoven web may be a spunbond web formed of continuous fibers. If the outer web is a spunbond web, the web may be (point-) bonded, by heat and/or pressure.

The inner nonwoven web may be a spunbond web formed of continuous fibers. If the inner web is a spunbond web, the web may be (point-) bonded, by heat and/or pressure. Alternatively, the inner nonwoven web may be a carded, air-through bonded layer formed of staple fibers.

At least 40% or at least 50%, or at least 70%, or all of the surface area of the elastic laminate does not comprise any additional material layers except the inner and outer web. The surface area is determined when the elastic laminate is stretched such that the inner and outer web are flattened out, or such that the inner and outer web are flattened out, in case further elongation is not possible without breaking and rupturing one of the inner and outer web. The extended portion of the outer web is folded over onto the respective other of the first and second web, thereby providing a region in the elastic laminate which has three webs overlaying each other, namely the inner web, the outer web, and the extended portion of the outer web. This extended portion of the outer web is not considered an "additional material layer". Likewise, elastic strands are not considered to be additional material layers. Additional material layers are materials which are provided in addition to the inner and outer web -and also in addition to the extended portion of the outer web- and having a significant s surface area, such as films, additional nonwoven webs, or paper.

To provide the inner and outer web with hydrophilicity, the inner and outer web may be treated with hydrophilic additives. Hydrophilic additives may be polypropylene and polyethylene polymers such as those available from Techmer PM (Clinton, TN, US) sold under the trade name of Techmer PPM15560; TPM12713, PPM19913, PPM 19441, PPM19914, and PM19668. Hydrophilic additives may include, ionic surfactants, cationic surfactants, amphoteric surfactants or mixtures thereof. Exemplary hydrophilic additives include 100410 AF PE MB marketed by Ampacet, Irgasuf HL560 commercially available from Ciba Speciality Chemicals Inc., Hydrosorb 1001 commercially available from Goulston Technologies Inc., Cirrasol PP682 commercially available from Uniqema, Stantex S 6327 commercially available from Cognis, Silastol PST, Silastol PHP26 commercially available from Schill & Seilacher, Silwet L-7608 commercially available from Momentive Performance Materials, silicone surfactant with a polyethylene oxide chain and molecular weight above 700 g/mol by the name Polyvel S-1416 or VW 315 commercially available from Polyvel Inc.

Elastic laminate used as a belt for a wearable article

The elastic laminate forms an elastic belt of a wearable article. The wearable article, such as a disposable pant, has a longitudinal direction extending from a front waist edge to a back waist edge, and a transverse direction extending perpendicular to the longitudinal direction.

The elastic belt has a front belt and a back belt. The front and back belt are discontinuous in the longitudinal direction of the wearable article. Left and right transverse edges of the front belt and the back belt are joined by side seams. Hence, the left transverse edge of the front belt is joined to the left transverse edge of the back belt by a first side seam and the right transverse edge of the front belt is joined to the right transverse edge of the back belt by a second side seam. Thereby, two leg openings and a continuous waist opening are formed. The continuous waist opening consists of the front waist edge and the back waist edge.

At the front and back waist edge, the elastic laminate comprises a fold over region. In this fold over region, the outer web of the elastic laminate is extended, i.e. it is longer than the inner web in the longitudinal dimension of the wearable article. The extended portion of the outer web is folded over the inner web, such that a portion of the elastic belt comprises the inner web sandwiched between the outer web and the extended portion of the outer web. The fold, i.e. the line where the outer web is folded over, forms the front and back waist edge of the wearable article. The extended folded over portion extends longitudinally in parallel with the longitudinal direction of the wearable article. It extends from the front and back waist edge, respectively, towards the crotch region of the wearable article. The extended folded over portion of the outer web extends from 20% to 70%, or from 30% to 70%, or from 40% to 70% of the total length of the side seams.

The total length of the side seams is determined from the waist opening to the leg opening along a straight line along the side seams. If the total length cannot be easily determined in the final product (i.e. the wearable article), the side seams may each be cut out from the wearable article but cutting left and right adjacent to the each of the side seams and then laying the cut out side seam portion flattened out on a table.

Therefore, the portion of the elastic laminate having the inner web sandwiched between the outer web and the extended, folded over portion of the outer web, constitutes a relatively large portion of the elastic laminate.

A portion of the inner web will be in direct contact with the skin of the wearer when the article is in use, i.e. it will form a portion of the innermost surface of the wearable article. The outer web may form at least a portion of the outermost surface of the wearable article, which will typically be in contact with the clothes of the wearer and which may frequently be touched by a caregiver. The extended, folded over portion of the outer web will be in direct contact with the skin of the wearer when the article is in use, i.e. it will also form a portion of the innermost surface of the wearable article.

The inner and outer web may be directly joined with each other over an area of from about 5% to about 50%. By "directly joined" what is meant is that the inner web and the outer web are directly secured to each other, e.g. by applying adhesive, ultrasonic, pressure, heat, or combinations thereof. The percentage of area of the inner and outer webs that are directly joined with each other may vary depending on the joining method for forming the elastic laminate, as discussed in further detail below. The inner and outer webs may be directly joined with each other over an area of from about 5% to about 50% to provide appropriate sweat management property, while also helping to maintain integrity as an elastic laminate.

The outer web of the elastic laminate may have a plurality of openings at an Opening Rate of from about 5% to about 50% according to the measurements herein. By further provide a certain opening area for the outer web, there is provided multiple moisture transport channels are provided which can contribute to an effective liquid removal and transport to the outer web. The transport channels may be driven by capillary force gradient, and enhanced exposure to outside the laminate away from the skin.

To provide a thickness gradient, the basis weight of the inner web may be not greater than the basis weight of the outer web. The inner web of the present invention is a nonwoven web which may have a basis weight of from about $5g/m^2$ to about $45g/m^2$, or from about $5g/m^2$ to about $35g/m^2$. The inner and outer web may have a fiber diameter of from $1\mu m$ up to $35\mu m$. The inner and/or outer web may also comprise nanofibers having a fiber diameter of below $1\mu m$. Fiber diameter, as

known in the industry, may also be expressed in denier per filament (dpf), which is grams / 9,000 meters of length of fiber. The inner web may be made by processes such as spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. The inner web may be made by biodegradable material or derived from renewable resources. Non-limiting examples of materials suitable for the inner web of the present invention include: 12-30gsm air-through carded nonwoven substrate made of PE/PET bi-component staple fiber, such as those available from Beijing Dayuan Nonwoven Fabric Co. Ltd. or Xiamen Yanjan New Material Co. Ltd., and 8-30gsm spunmelt nonwoven substrate comprising PP monofilament or PE/PP bi-component fibers, such as those available from Fibertex or Fitesa.

The inner web is hydrophilic. As the outer web comprises an extended portion which is folded over the inner web, the hydrophilicity of the inner web can help transport away from the skin of the wearer through the extended, folded portion of the outer web (which is no at least partly in direct contact with the wearer's skin).

The inner web may optionally have a plurality of openings at an Opening Rate of from about 5 % to about 30 %, or from about 5 % to about 15 %, or from about 6 % to about 8 %, or from about 7 % to about 15 %, or from about 9 % to about 25 %, and an Effective Opening Area of from about $0.1 \text{ mm}^2$ to about $25 \text{ mm}^2$, or from about $0.1 \text{ mm}^2$ to about $10 \text{ mm}^2$, or from about $0.5 \text{ mm}^2$ to about $4 \text{ mm}^2$, or from about $4.0 \text{ mm}^2$ to about $8 \text{ mm}^2$, or from about $7 \text{ mm}^2$ to about $15 \text{ mm}^2$, according to the measurements herein.

The outer web of the elastic laminate may have a basis weight of from about $10\text{g/m}^2$ to about $45\text{g/m}^2$, or from about $10\text{g/m}^2$ to about $35\text{g/m}^2$, and may be adjusted such that the basis weight of the inner web is greater, equal or smaller than the basis weight of the outer web. If the outer web is the first web, the outer web may be made by processes such as spunbond, spunlace, carded or air-laid; and may comprise fibers and/or filaments made of polypropylene (PP), polyethylene (PE), polyethylene phthalate (PET), polylactic acid/polylactide (PLA) or conjugate fibers (such as PE/PET, PE/PP, PE/PLA) as well as natural fibers such as cotton or regenerated cellulosic fibers such as viscose or lyocell. Also, if the outer web is the first web, the outer web may be made by biodegradable material, or derived from renewable resources.

Both inner web and outer web are hydrophilic. They may have equal or similar vertical wicking height after 60 seconds according to the Vertical Wicking Height method set out herein below. They may have both a wicking height of, for example, below 5 mm after 60 seconds according to the Vertical Wicking Height method.

Exemplary material for the outer web include: air-through carded nonwoven having a thickness of at least about $50 \mu\text{m}$, or at least about $80 \mu\text{m}$, or at least about $200 \mu\text{m}$. The thickness may be less than $2000 \mu\text{m}$, or less than $1500 \mu\text{m}$, or less than $1250 \mu\text{m}$. Such material may provide a soft lofty feeling to the garment-facing web. Suitable for the outer web of the present invention are air-through carded nonwoven material made of co-centric bicomponent fiber, crimping fiber made through core eccentric bicomponent filament or side by side bicomponent filament. Non-limiting examples of materials suitable for the outer web include: $12 \text{ g/m}^2$ to $45\text{g/m}^2$ air-through carded nonwoven substrate comprising PE/PET bi-component fibers, such as those available from Beijing Dayuan Nonwoven Fabric Co. Ltd. or Xiamen Yanjan New Material Co. Ltd., and 8-45gsm spun melt nonwoven substrate comprising PP monofilament or PE/PP bi-component fibers, such as those available from Fibertex or Fitesa.

The basis weight and material thickness of the inner and outer webs herein is related to materials obtained from a finished product according to the "Preparation for Thickness and Basis Weight" below and measured by "Base caliper method - ASTM D 654 Standard Test Method for Thickness of Paper and Paper Board" with modification of the loading to 500Pa, and by "Basis weight - ASTM D 756 Practice for Determination of Weight and Shape Changes of Plastics Under Accelerated Service Conditions", respectively.

The outer web may have a plurality of openings at an Opening Rate of from about 5% to about 50%, or from about 5% to about 30%, or from about 7% to about 15%, or from about 9% to about 25%, and an Effective Opening Area of from about $0.1\text{mm}^2$ to about $25\text{mm}^2$, or from about $0.4\text{mm}^2$ to about $2.0 \text{ mm}^2$, of from about $1.0 \text{ mm}^2$ to about $5 \text{ mm}^2$, or from about $4.0 \text{ mm}^2$ to about $8 \text{ mm}^2$, or from about $7 \text{ mm}^2$ to about $15 \text{ mm}^2$, according to the measurements herein.

Alternatively, or in addition, the inner web may haves a plurality of openings at an Opening Rate of from about 5% to about 50%, or from about 5% to about 30%, or from about 7% to about 15%, or from about 9% to about 25%, and an Effective Opening Area of from about $0.1\text{mm}^2$ to about $25\text{mm}^2$, or from about $0.1\text{mm}^2$ to about $10\text{mm}^2$, or from about $0.4\text{mm}^2$ to about $2.0 \text{ mm}^2$, or from about $0.5\text{mm}^2$ to about $4\text{mm}^2$, of from about $1.0 \text{ mm}^2$ to about $5 \text{ mm}^2$, or from about $4.0 \text{ mm}^2$ to about $8 \text{ mm}^2$, or from about $7 \text{ mm}^2$ to about $15 \text{ mm}^2$, according to the measurements herein

For either the outer web and/or the inner web, the openings may be apertures, slits, or the like. Preferably, the openings are apertures. If the inner and outer web have openings, the openings of the inner web may be congruent with the openings of the outer web. Alternatively, if the inner and outer webs have openings, the openings of the inner web may not be congruent with the openings of the outer web. In a still further alternative, if the inner and outer webs have openings, some of the openings in the inner web may be congruent with the openings in the outer web, while the remaining openings in the inner web may not be congruent with the openings of the outer web.

The openings in the inner and/or outer web may be apertures having an aspect ratio of less than about 5. The aspect ratio of an opening is determined as such. The greatest dimension of the opening is measured, wherein the direction of the greatest dimension defines the first axis. The line perpendicular to the first axis is defines the second axis. The dimension of the opening along the second axis is measured and defined the cross dimension. The aspect ratio is the greatest dimension divided by the cross dimension.

The openings may be made by female-male hot pin process, hole punching process, hydroentanglement process using water jets and a screen to create holes, and combinations thereof. The openings may be made by creating a plurality of weakened locations by heat or pressure followed by incrementally stretching, causing said nonwoven web to rupture at the weakened locations such as described in US Patent 5,628,097. Such rupturing method may be particularly useful for nonwovens using spunbonded fibers and meltblown fibers. The openings may be three-dimensional, nonhomogeneous, unaligned and forming a pattern as described in PCT Publication WO 2016/73712.

The inner web or the outer web may be devoid of openings (alternatively, both layers may be devoid of openings). Namely, the combination of the inner web and the outer web may provide a Relative Opening Rate of 100%, less than 100%, or less than about 50%, or less than about 15% according to the measurements herein. What is meant by Relative Opening Rate is the percentage of opening of the elastic laminate which matches the opening of the outer web. When there is only opening in the outer web, the Relative Opening Rate is 0%, while when the openings of the outer web and the inner web completely match, the Relative Opening Rate is 100%. In the present invention, the inner web may optionally have openings, and when so, the pattern and density of the openings may be changed so as to completely or partially match with those of the outer web.

The inner web and the outer web may be directly joined to each other over an area of from about 5% to about 50% by any means known in the art, such as by applying adhesive, ultrasound, pressure, or heat, for providing the elastic laminate of the present invention. The inner and outer web may be at least partially directly joined by adhesive agent. When adhesive agent is used for joining the inner and outer webs, the area in which adhesive agent is applied between the inner and outer webs is considered as area in which the webs are directly joined. When using adhesive agent as a joining means, the adhesive agent may be applied intermittently, such as in spiral pattern. Alternatively or additionally, the adhesive agent may be applied by a slot coat pattern for sake of better process control, wherein the area in which adhesive agent is applied is from about 5% to about 50%, or from about 5% to about 40%, or from about 5% to about 30% of the laminate planar area. Alternatively or additionally, the inner and outer webs may be at least partially directly joined by means which directly join the fibers of the inner and outer webs, such as by heat, pressure, or ultrasound.

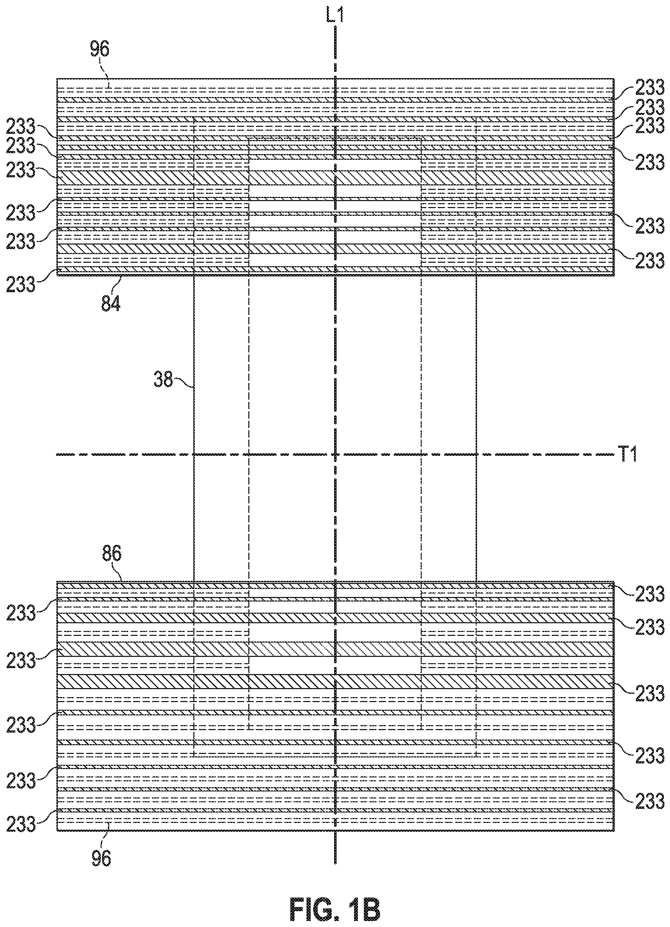

The elastic laminate of the present invention may have an elongation rate of at least about 110% in at least one direction. Elasticity may be imparted by laminating an elastic body between the inner web and the outer web. The elastic body may preferably be a plurality of elastic strands, but may alternatively be elastic ribbons, or an elastic sheet. The webs and elastic bodies may be at least partially joined by means selected from the group consisting of; adhesive agent, heat, pressure, ultrasound, and combinations thereof. Referring to Figure 1B, adhesive may be applied for joining the elastic bodies to the outer and/or inner web, and further applied via a slot coat in a pattern of panel adhesive 233 for joining the outer layer and the inner web. Alternatively or additionally, the elastic bodies may be joined by deforming the inner and/or outer web contacting the elastic body via ultrasound or heat, to anchor the elastic body against the inner and/or outer web. Though less preferred, the elastic body may be an elastic sheet; wherein ultrasound is applied at a certain energy level to the inner web, outer web, and elastic sheet, combined, such that the fibers of the inner web and the outer web come into direct contact with each other. These directly joined areas of fibers are also considered as area in which the inner and outer webs are directly joined.

Elastic laminates obtained by any of the aforementioned joining methods need not be embossed, or mechanically activated, beyond the force needed to at least partially directly join the layers. Thus, the elastic laminate may be economically made. The directly joined area may be measured by stretching the elastic laminate to an uncontracted condition, suitably with a force of 25N, and observing the planar area where the inner and outer layers are directly joined.

The wearable article

The present invention relates to a wearable article comprising an elastic laminate that forms the belt of the wearable article. The elastic laminate may form at least a part of a wearable article that is in direct contact with the skin.

The wearable article may be a pant. An exemplary pant is described in PCT Publication WO 2006/17718A. The pant may comprise a central chassis 38 to cover the crotch region of the wearer when the article is worn, a front belt 84 and a back belt 86 (hereinafter may be referred to as "front and back belts") comprising the elastic laminate of the present invention, the front and back belts 84, 86 forming a discrete ring-like elastic belt 40 (hereinafter may be referred to as "waist belt") extending transversely defining the waist opening. A discrete ring-like elastic belt described a belt wherein the front belt and the back belt are discontinuous in the longitudinal direction of the wearable article. A wearable article with a discrete ring-like belt may be advantageous versus a uni-body type pant wherein the central chassis 38 is continuous with the front and back belt 84, 86, wherein the leg openings are continuously formed (not shown) in that the central chassis 38 has better breathability, thus providing better sweat management for the entire wearable article.

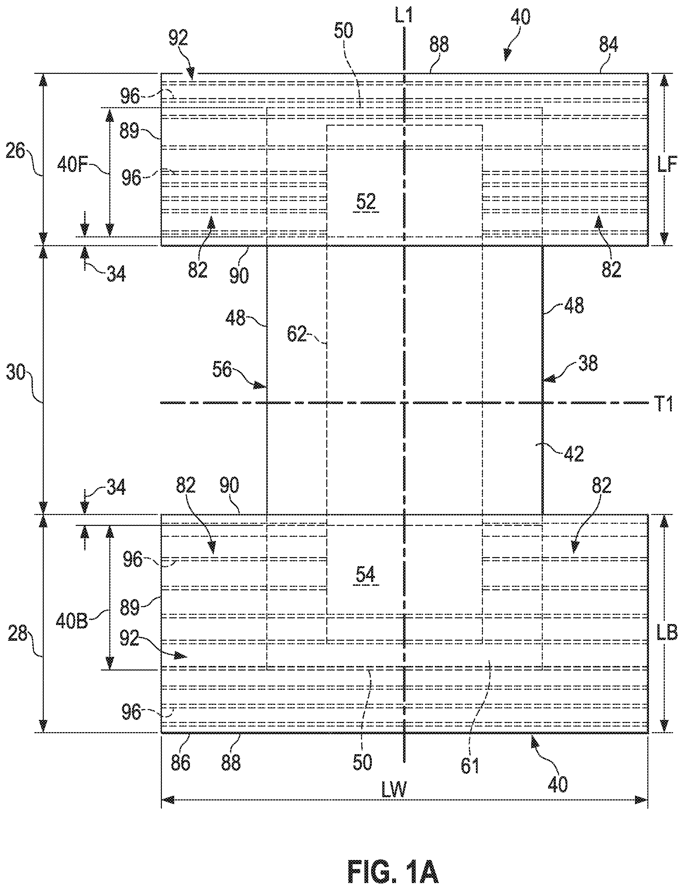

Figure 1A is a perspective view of an example for a wearable article of the present invention of the pant with the seams un-joined and in its flat uncontracted condition showing the garment-facing surface. The wearable article has a longitudinal centerline L1 which also serves as the longitudinal axis, and a transverse centerline T1 which also serves as the transverse axis. The longitudinal direction of the wearable article extends in parallel with the longitudinal centerline L1 and the transverse direction of the wearable article extends in parallel with the transverse centerline T1. The wearable article has a body facing surface, a garment facing surface, a front waist region 26 formed by the front belt, a back waist region 28 formed by the back belt, a crotch region 30 extending between the front belt and the back belt in the longitudinal direction fo the wearable article, and side seams which join the front waist region 26 and the back waist region 28 to form two leg openings and a waist opening. The front and back belts 84, 86 and the central chassis 38 jointly define the leg openings.

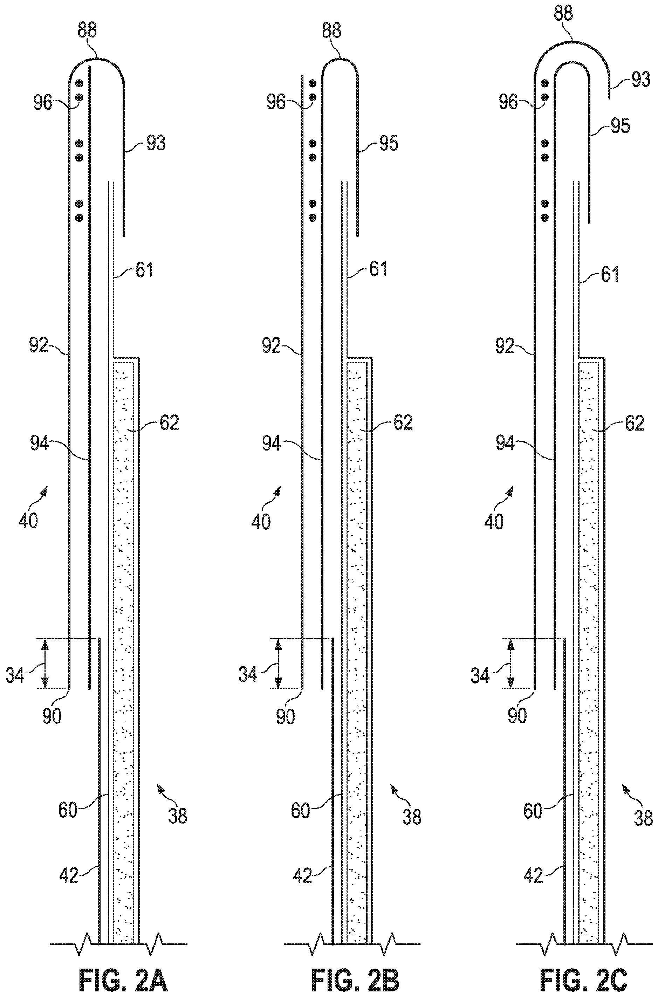

As exemplarily shown in Figures 1A and 2A-2C, the central chassis 38 may comprise a backsheet 60 and an outer cover layer 42 for covering the outer surface of the backsheet 60. The backsheet 60 may be a water impermeable film. At least a portion of or the entirety of the outer cover layer 42 may be the elastic laminate of the present invention. The central chassis 38 may contain an absorbent core 62 for absorbing and containing body exudates disposed on the central chassis 38. As exemplified in in Figure 1A, the central chassis 38 may have a generally rectangular shape, left and right longitudinally extending side edges 48 (hereinafter may be referred to as "side edge") and front and back transversely extending end edges 50 (hereinafter may be referred to as "end edge"). The central chassis 38 also has a front waist panel 52 positioned in the front region 26 of the wearable article, a back waist panel 54 positioned in the back region 28, and a crotch panel 56 between the front and back waist panels 52, 54 in the crotch region 30. The center of the front belt 84 is joined to a front waist panel 52 of the central chassis 38, the center of the back belt 86 is joined to a back waist panel 54 of the central chassis 38, the front and back belts 84, 86 each having a left side panel and a right side panel 82 where the central chassis 38 does not overlap. The central chassis 38 may comprise one or more leg cuffs per side for gasketing the leg opening.

The ring-like elastic belt 40 of the pant of the present invention acts to dynamically create fitment forces and to distribute the forces dynamically generated during wear. The proximal edge 90 is located closer than the distal edge 88 relative to the crotch panel 56 of the central chassis 38. Each leg opening may be provided with elasticity around the perimeter of the leg opening. For the belt-type pant, the elasticity around the leg opening may be provided by the combination of elasticity from the front belt 84, the back belt 86, and any from the central chassis 38 (e.g. by elastic strands comprised by the leg cuffs). The leg cuffs may be provided adjacent the left and right longitudinally extending side edges of the central chassis 38.

The front belt 84 and back belt 86 of the pant are configured to impart elasticity to the belt 40. The front belt 84 and the back belt 86 are each formed by the present elastic laminate and may comprise a plurality of elastic bodies 96, such as elastic strands, running in the transverse direction and being provided between the inner web 94, and the outer web 92 of the elastic laminate.

The elastic bodies, such as elastic strands, may not be provided between the inner web and the extended, folded over portion of the outer web.

The extended, folded over portion of the outer web is preferably provided around the complete waist opening 88 of the wearable article.

When the central chassis 38 contains an absorbent core, some or all of the areas of the front or back belt 84, 86 overlapping the absorbent core may be made devoid of elasticity. Referring to Figure 1A, areas of the front waist panel 52 and back waist panel 54 in which the elastic bodies 96 of the overlapping elastic laminate are deactivated are shown in blank. For example, as seen in the back belt 86, the elastic bodies 96 overlapping the absorbent material non-existing region 61 and toward the distal edges of the absorbent core 62 may be disposed in active elasticity for good fit of the central chassis 38. This may be advantageous in preventing leakage.

Providing an extended, folded over portion 95, 93 of the outer web is advantageous for avoiding the waist opening 88 ending in sharp edges of the front or back belt 84, 86. Further, any elastic bodies 96 in the front or back belt 84, 85 may be disposed at least about 2 mm away, or from about 5 mm to about 9 mm away from the waist opening, to avoid the waist opening to be sharp, and also to ensure that any elastic body is not accidentally exposed during manufacture or use. The extended, folded over portion 95, 93 of the outer web extends toward the proximal edge of the belt (i.e. towards the crotch region). There may be an overlap between the extended, folded over portion of the outer web and the central chassis 38 by at least about 10 mm, or by at least about 15 mm, to secure integrity between the front and or back belt 84, 86 and central chassis 38. The overlap may be less than 40 mm, or less than 30 mm.

Referring to Figure 1A, the transverse width LW of the back belt 86 in the uncontracted condition may be the same as the transverse width of the front belt 84 of the same condition. Such an article may be economically made. The longitudinal length LB of the back belt 86 between the back distal edge 88 (forming the back waist edge) and the back proximal edge 90 along its entire width LW of the back belt 86 may be approximately the same as the longitudinal length LF of the front belt 84 between the front distal edge 88 (forming the front waist edge) and the front proximal edge 90. In such configuration, the side seams close the front and back belt 84, 86 transverse edges 89 are of the same length for forming the article. Such an article may be economically made. The back belt 86 may have a greater longitudinal length LB between the back distal edge 88 (forming the back waist edge) and the back proximal edge 90 along its entire width LW of the back belt 86 in the transverse direction than the longitudinal length LF of the front belt 84 between the front distal edge 88(forming the front waist edge) and the front proximal edge 90. In such configuration, when the wearable article is assembled to form the waist opening and the leg openings, the wearable article is folded along the transverse centerline T1 such that the front distal edge 88 (i.e. with the front waist edge) is aligned with the back distal edge 88 (i.e. with the back waist edge). The front transverse edge 89 is also aligned with a portion of the back transverse edge 89. Then the front belt 84 and the back belt 86 are joined at the front and back transverse edges 89 at the seams. The front and back proximal edges 90, however, may not be aligned to one another. The back proximal edge 90 may be disposed longitudinally closer than the front proximal edge 90 relative to the transverse center line T1 such that the proximal portion of the back side panel 82 extends toward the crotch panel 56 of the central chassis 38 beyond the front proximal edge 90. The transverse edge of the proximal portion of the back side panel 82 may not be joined to anywhere and free from attachment. Thus, the proximal portion of the back side panel 82 provides a buttock cover.

Referring to Figure 1A and 2A-2C, the front and back belts 84, 86 are discontinuous with one another in the crotch region 30, such that the outer cover layer 42 is the garment-facing surface in the crotch region 30. The outer cover layer 42 may extend only partly in the longitudinal direction of the front waist panel 52 and the back waist panel 54 to leave the distal parts of the front waist panel 52 and the back waist panel 54 free of the outer cover layer 42. Namely, the longitudinal length of the outer cover layer 42 may be longer than the longitudinal length of the crotch panel 56 (i.e. the longitudinal extension between the proximal edges of the front and back belt) and shorter than the longitudinal length of the backsheet 60. By such configuration, the distal parts of the front waist panel 52 and the back waist panel 54 are devoid of the outer cover layer 42, providing better breathability and sweat management for the elastic belt 40. Further, this may provide cost saving of the outer cover layer 42 material. Accordingly, looking at the layers of elements between the garment facing surface and the backsheet 60 of the center chassis 38, there exists a transitional region 34 disposed on the front and back waist panel 52, 54 where the outer cover layer 42 is present. The longitudinal length of the transitional region 34 may be made as short as possible, for example, less than about 20 mm, or less than about 15 mm, or less than about 10 mm. Further, adhesive may be applied on the entire area of the transitional region 34, or the entire area leaving no more than up to 5 mm, in the longitudinal direction, from the distal edge of the transitional region 34. For providing attractive artwork for a wearable article in an economical manner, printing may be provided on the garment facing side of the backsheet 60. By providing the transitional region 34 as short as possible, applying adhesive to the transitional region 34 to enhance transparency, or simply avoiding displaying artwork in the transitional region 34, compromised appearance of the artwork over different layers of material between the artwork and the observer may be avoided. Referring to Figure 1A, artwork on the backsheet 60 may be printed in regions 40F and/or 40B.

The articles of the present invention provide improved sweat management properties, are easy to apply and comfortable to wear, while being economic to make.

Bio-based materials

The elastic laminate may comprise a bio-based content value from about 10% to about 100% using ASTM D6866-10, method B, or from about 25% to about 75%, or from about 50% to about 60%.

The inner web and outer web of the elastic laminate may comprise a bio-based content value from about 10% to about 100% using ASTM D6866-10, method B, or from about 25% to about 75%, or from about 50% to about 60%.

In order to apply the methodology of ASTM D6866-10 to determine the bio-based content of a single component material (i.e. the elastic laminate), that material is isolated and cleaned such that the resulting specimen reflects the constituent starting material as closely as possible. For example, if a nonwoven component of an elastic nonwoven laminate is of interest, the laminate is deconstructed (with elastic strands removed) and the nonwoven layer is washed with an appropriate solvent so as to remove any residual adhesive present. In order to apply the methodology of ASTM D6866-10 to an sample assembly of two or more materials of differing or unknown compositions, the sample is homogenized by grinding the material into particulate form (with particle size of about 20 mesh or smaller) using known grinding methods (such as with a Wiley grinding mill). A representative specimen of suitable mass is then taken from the resulting sample of randomly mixed particles.

Validation of Polymers Derived from Renewable Resources

A suitable validation technique is through 14C analysis. A small amount of the carbon dioxide in the atmosphere is radioactive. This 14C carbon dioxide is created when nitrogen is struck by an ultra-violet light produced neutron, causing the nitrogen to lose a proton and form carbon of molecular weight 14 which is immediately oxidized to carbon dioxide. This radioactive isotope represents a small but measurable fraction of atmospheric carbon. Atmospheric carbon dioxide is cycled by green plants to make organic molecules during photosynthesis. The cycle is completed when the green plants or other forms of life metabolize the organic molecules, thereby producing carbon dioxide which is released back to the atmosphere. Virtually all forms of life on Earth depend on this green plant production of organic molecules to grow and reproduce. Therefore, the 14C that exists in the atmosphere becomes part of all life forms, and their biological products. In contrast, fossil fuel based carbon does not have the signature radiocarbon ratio of atmospheric carbon dioxide.

Assessment of the renewably based carbon in a material can be performed through standard test methods. Using radiocarbon and isotope ratio mass spectrometry analysis, the bio-based content of materials can be determined. ASTM International, formally known as the American Society for Testing and Materials, has established a standard method for assessing the bio-based content of materials. The ASTM method is designated ASTM D6866-10.

The application of ASTM D6866-10 to derive a "bio-based content" is built on the same concepts as radiocarbon dating, but without use of the age equations. The analysis is performed by deriving a ratio of the amount of organic radiocarbon (14C) in an unknown sample to that of a modern reference standard. The ratio is reported as a percentage with the units "pMC" (percent modern carbon).

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, for example, it would give a radiocarbon signature near 54 pMC (assuming the petroleum derivatives have the same percentage of carbon as the soybeans).

A biomass content result is derived by assigning 100% equal to 107.5 pMC and 0% equal to 0 pMC. In this regard, a sample measuring 99 pMC will give an equivalent bio-based content value of 92%.

Assessment of the materials described herein can be done in accordance with ASTM D6866. The mean values quoted in this report encompasses an absolute range of 6% (plus and minus 3% on either side of the bio-based content value) to account for variations in end-component radiocarbon signatures. It is presumed that all materials are present day or fossil in origin and that the desired result is the amount of biobased component "present" in the material, not the amount of biobased material "used" in the manufacturing process.

**Test methods**

Contact Angle Test Method

A rectangular specimen of the web, measuring 1 cm x 2 cm, is removed from the elastic laminate of a wearable article so as not to disturb the structure of the material. The specimen has a length of (2 cm) aligned parallel to the longitudinal centerline of the article. The specimen of interest may be separated from the other components of the wearable article such as the inner web or the outer web of the elastic laminate, elastic bodies between the inner and outer web, or backsheet or any other material by techniques such as applying freeze spray, or other suitable methods that do not permanently alter the properties of the web. The extracted web specimen is conditioned at a temperature of $23\pm2$ °C and a relative humidity of $50\pm10$ % for at least 24 hours. The specimen is handled gently throughout by the edges using forceps and is mounted flat on an SEM specimen holder using double-sided tape. Multiple specimens are prepared in similar fashion as needed to accumulate the requisite number of individual measurements.

The specimen is sprayed with a fine mist of water droplets generated using a small hobby air-brush apparatus. The water used to generate the droplets is distilled deionized water with a resistivity of at least 18 MQ-cm. The airbrush is adjusted so that the droplets each have a volume of about 2 pL. Approximately 0.5 mg of water droplets are evenly and gently deposited onto the specimen. Immediately after applying the water droplets, the mounted specimen is frozen by plunging it into liquid nitrogen. After freezing, the sample is transferred to a Cryo-SEM prep chamber at -150 °C, coated with Au/Pd for 2 minutes, and transferred into Cryo-SEM chamber at -150 °C. A Gatan Alto 2500 Cryo-SEM prep chamber or equivalent instrument is used as preparation chamber. A Hitachi S-4700 Cryo-SEM or equivalent instrument is used to obtain high-resolution images of the droplets on the fibers. Droplets are randomly selected, though a droplet is suitable to be imaged only if it is oriented in the microscope such that the projection of the droplet extending from the fiber surface is approximately maximized. The contact angle between the droplet and the fiber is determined directly from the image.

The above procedure is used on the inner web to determine the Inner Web Contact Angle. Ten droplets, located on the inner web, are imaged from which 20 contact angle measurements are performed (one on each side of each imaged droplet), and the arithmetic mean of these 20 contact angle measurements is calculated and reported as the Inner Web Contact Angle to the nearest 0.1 degree.

The above procedure is used on the outer web to determine the Outer Web Contact Angle. Ten droplets, located on the outer web, are imaged from which 20 contact angle measurements are performed (one on each side of each imaged droplet), and the arithmetic mean of these 20 contact angle measurements is calculated and reported as the Outer Web Contact Angle to the nearest 0.1 degree.

Residual Fluid Method

The test was carried at a temperature of $23°C \pm 2°C$ and a relative humidity of $50\% \pm 10\%$.

The specimen was conditioned at a temperature of $23°C \pm 2°C$ and a relative humidity of $50\% \pm 10\%$ for at least 24 hours.

Ca. 0.24g saline (0.9% wt. of NaCl in water solution) are loaded onto a glass Petri dish (having a diameter of 9cm), actual, exact amount of saline, $M_{fluid}$, is determined via subtracting the dry glass Petri dish weight ($M_{P,D}$, prior to the application of the fluid) from the glass Petri dish weight with ca. 0.24g fluid ($M_{P,L}$):

$$M_{fluid} = M_{P,L} - M_{P,D}$$

The sample is laid over the fluid gently, without any pressure, other than the sample weight itself. Web 1 is placed in contact with the fluid. After 1 minute of contact, the sample is removed and the final weight of Petri dish is recorded ($M_{P,F}$): the residual fluid on Petri dish is determined via subtracting the dry glass Petri dish weight, prior to the application of the fluid, from the final glass Petri dish weight:

$$M_{Residual} = M_{P,F} - M_{P,D}$$

The % of Residual Fluid on the Petri dish is calculated as below

$$\% \text{ Residual Fluid on Petri dish} = M_{Residual} / M_{fluid} \times 100$$

expressed in % 3 replicates are performed with each sample.

Relative Opening Rate Test Method

Relative Opening Rate is the opening rate of the outer web and the inner web combined, compared to the opening rate of the outer web. Before preparation of specimen, the relationship of openings from the outer web and the inner web as the elastic laminate are observed. If the inner web has no openings, the Relative Opening Rate is determined as 0%. If the inner web has openings and the openings from the outer web and the inner web appear to completely or substantially match, the specimen is arranged as A). If the inner web has openings and the openings from the outer web and the inner web appear to partially match or not match, the specimen is arranged as B).

A) The elastic laminate is treated according to the Preparation of specimen above to obtain the inner and outer webs. The inner and outer webs are overlayed such that the openings match each other as much as possible. The overlayed specimen is sent for measurement.
B) The elastic laminate is treated according to the Preparation of specimen above to obtain the inner and outer webs. The inner and outer webs are overlayed in 2 different degrees of overlap of openings, one which has the openings matched as much as possible, and another which has the openings unmatched as much as possible. The 2 types of overlayed specimen are sent for measurement, respectively. The average Relative Opening Rate of the 2 types of overlayed specimen is obtained.

The overlayed specimen are measured in the same manner as specified under "2. Effective Opening Area and Opening Rate" to obtain the Opening Rate of the overlayed specimen.

The Relative Opening Rate is obtained as such. When there is a complete match between the inner web and the outer web, the Relative Opening Rate is 100%.

Relative Opening Rate (%) = Opening Rate of overlayed specimen / Opening Rate of outer web $\times$ 100

Mean Flow Pore Size

Mean Flow Pore Size of nonwoven is characterized by the gas-liquid displacement method according to ASTM F316, using a capillary flow porometer such as Porolux™ 100 NW (Porometer N.V., Belgium). The porometry measurement follows the Young-Laplace equation, $P = 4 * \gamma * \cos(\theta) / D$, where D is the pore size diameter, P is the pressure measured, $\gamma$ is the surface tension of the wetting liquid, and $\theta$ is the contact angle of the wetting liquid with the sample. The procedures are the following:

1) Wet the specimen with a liquid of low surface tension and low vapor pressure, for example, a commercial wetting liquid Porefil (Prorometer N.V., Belgium) with surface tension of 16 mN/m. Consequently, all pores are filled with the liquid.
2) An inert gas is used to displace wetting liquid from pores and gas flow rate is normally measured using flow meters. The liquid is blown out of the specimen by gradually increasing the gas pressure. When further increasing the pressure, gas flows through small pores until all the pores are emptied. Record the gas pressure and gas flow rate when liquid is being expelled.
3) After the wet run, the measurement of the same sample in dry state is carried out.
4) The pore size parameters are calculated by comparing the pressure-flow rate curves from the wet run and dry run according to ASTM F316.

Vertical Wicking Height Method

The Vertical Wicking Height Method is used to determine the Vertical Wicking Height of a web available as roll-stock raw material or a web removed from the elastic laminate of an article. The method is performed according to the general principles described in EDANA method 10.4 (02), section 6 "LIQUID WICKING RATE". Five test pieces, having dimensions of 250 mm in length and 25 mm in width, are tested.

If the web is available as roll stock, a specimen having a width of $25 \pm 1$ mm and a length of $250 \pm 1$ mm, wherein the length is measured parallel to the machine direction of the material.

If the web is not available as roll stock, it is removed from an article. To remove the elastic laminate from the article the following procedure is used. It is identified the elastic laminate of the wearable article and cut a piece of elastic laminate, having a width of $25 \pm 1$ mm and a length of $250 \pm 1$ mm, wherein the length is referred to the state of the elastic laminate stretched until the material is flat, i.e. the laminate shows no wrinkles. The long direction of the specimen is parallel from other components of the wearable article via applying a cryogenic freeze spray without damaging the material properties. Once the elastic laminate is removed, the inner web and outer web are carefully separated without permanently altering the properties of the inner and outer web. Four additional like specimens from like absorbent articles are prepared for a total of five specimens. At the end of this sample preparation procedure, five like specimens of inner web and five like specimens of outer web result.

The web specimens are conditioned for 24 h at 23 °C and 50 % relative humidity. Each specimen is conditioned for 24 h at 23 °C and 50 % relative humidity and then analyzed Each analysis is carried at 23 °C and 50 % relative humidity. The fluid used is 0.9 % (mass per mass) NaCl water solution.

Each specimen is hung vertically with the bottom punctured by two stainless steel needles and attached to an additional clamp ($30 \pm 1$ g) to keep the sample extended. The two needles are inserted in the nonwoven within the bottom 15 mm of the length of the nonwoven. The two needles are inserted parallel to the direction of the 25mm width of the specimen, with a distance of about 8mm in between the two needles. The clamp is clamped in between two needles. The stainless steel needle is at least as wide as the strip and has a diameter sufficient to prevent the clamp from sliding over the needle. The clamp is 25 mm wide.

The bottom of the specimen is dipped into the fluid reservoir at beginning, and the vertical wicking height is measured at time points of interest from the point at which the web intersects the free liquid surface. Each specimen is measured in this way, and for each specimen, the height of capillary rise of liquid at 10 s, 30 s, and 60 s is recorded the nearest 1 mm. If the capillary rise is not a uniform straight line, the highest distance, farthest from the free liquid surface, is measured.

The arithmetic means of the capillary rise among the five specimens of inner web corresponding to each of 10 sec, 30 sec and 60 sec are calculated and are reported as the Inner Web Vertical Wicking Height after 10 sec, Inner Web Vertical Wicking Height after 30 sec, and Inner Vertical Wicking Height after 60 sec, respectively, to the nearest 1 mm. The arithmetic means of the capillary rise among the five specimens of outer web corresponding to each of 10 sec, 30 sec and 60 sec are calculated and are reported as the Outer Web Vertical Wicking Height after 10 sec, Outer Web Vertical Wicking Height after 30 sec, and Outer Vertical Wicking Height after 60 sec, respectively, to the nearest 1 mm.

Surface Area /Volume Test Method

The Surface Area Per Volume Method uses analysis with a scanning electron microscope (SEM) to determine the surface area per volume of a web, such as the inner and outer web. SEM images containing front-face views and/or cross-sections of fibers are used to measure the perimeter per cross sectional area of individual fibers, which is deemed to correspond directly to the surface area per volume ratio of these same fibers, from which surface area per volume present in each layer is determined.

A rectangular specimen of the web, measuring 1 cm x 2 cm, is removed from the elastic laminate of a wearable article taking care not to disturb the structure of the material. The specimen has a length of (2 cm) aligned with a longitudinal centerline of the wearable article. The specimen of interest may be separated from the other components of the wearable article such as the inner web or the outer web, respectively (depending which web is to analyzed), elastic bodies between the inner and outer web, or backsheet or any other material by techniques such as applying freeze spray, or other suitable methods that do not permanently alter the properties of the web. The extracted web specimen is conditioned at a temperature of $23\pm2$ °C and a relative humidity of $50\pm10$ % for at least 24 hours. The specimen is handled gently throughout by the edges using forceps and is mounted flat on an SEM specimen holder using double-sided tape. Multiple specimens are prepared in similar fashion as needed to accumulate the number of measurements. In instances in which cross sectional analysis is performed, as described below, a new single-edged razor blade (such as 0.009" (0.22 mm) thick surgical carbon steel razor blade (part number 55411-050 from VWR, Radnor, PA, USA, or equivalent) is used to cross-section the specimen prior to mounting in the 2-cm dimension, and one of the fresh cross-sectional faces is subsequently analyzed in the SEM. Prior to introduction into the SEM, each specimen is sputtered with gold or a palladium compound to avoid electric charging and vibrations of the fibers in the electron beam

A scanning electron microscope (SEM) is used to analyze the top view and cross section of the fibers.

A magnification of 500 to 10,000 times is chosen such that the ratio of target fiber perimeter to Horizontal Field Width (HFW) is bigger than 0.5. Secondary electron images are acquired with a standard Everhart-Thornley detector.

An initial cross-sectional SEM image of the web of interest is capture. If fibers present have a circular cross-section, then fiber-width measurements from top view images can be used as diameter, and corresponding perimeters (circumferences) are calculated for each diameter assuming circular cross sections. No fiber is measured more than once, and surface area to volume of each fiber measured is recorded as the circumference-to-area ratio at this point of measurement, i.e. $\pi D/ ((\pi D2)/4) = 4/D$, where D is the measured diameter of the fiber. If fibers present in the web do not have a circular cross-section, the area and perimeter for each fiber analyzed are directly measured from SEM cross-sectional web images. The use of image analysis software, such as Image J (NIH, Bethesda, MD, USA, or equivalent) may be used to aid in the accurate and facile measurement of cross-sectional perimeters. The perimeter and area of each cross section measured is recorded, as is the ratio of perimeter to area for each cross section.

If the web of interest exhibits a gradient in fiber size and/or shape, each distinct fibrous layer is separately characterized.

At least 100 measurements of individual fibers are performed in the web of interest (e.g. the inner web or outer web). The arithmetic mean of the ratios of cross-sectional perimeter to area recorded among fibers in each layer present is calculated, and this is reported as the surface area per volume in the web of interest. The surface area to volume ratio is reported in 1/mm to the nearest 0.1 1/mm.

Fiber Diameter Method

The average equivalent fiber diameter of each of one or more distinct fiber(s) layer present in a web is done following the Surface Area Per Volume Method. Once the average surface area per volume (SApV) has been determined for a given web, the average equivalent diameter for that web is calculated as 4/SApV. The average equivalent fiber diameter is reported in micrometers ($\mu$m), to the nearest 0.1 $\mu$m.

**Examples**

The following four nonwovens webs were used to form the elastic laminates described below:

Nonwoven 1: Spunbond nonwoven (SSS, i.e. three identical spunbond layers); 100% polypropylene; fiber diameter of about 15 $\mu$m; basis weight of 15 gsm. The nonwoven has been supplied by Fibertex under the tradename A10150AH.

Nonwoven 2: Carded air-through bonded nonwoven, bicomponent PE/PET 50% PET (core of bicomponent fibers), 50% polyethylene (shell of bicomponent fibers), fiber diameter of about 14.3$\mu$m; basis weight of 20 gsm. The nonwoven has been supplied by Dayuan under the tradename FJ206.

Nonwoven 3: Spunbond nonwoven (SSS, i.e. three identical spunbond layers); 100% polypropylene; fiber diameter of about 16.2 μm; basis weight of 15 gsm. The nonwoven has been supplied by Fibertex under tradename A20150KV.

Nonwoven 4: Carded air-through bonded nonwoven, bicomponent PE/PET 50% PET, 50% polyethylene, fiber diameter of about 14.8μm; basis weight of 22 gsm. The nonwoven has been supplied by Yanjan. Under the tradename Z05X-22.

The vertical wicking height of each of Nonwovens 1 to 4 was tested according to the test method set out above. The vertical wicking height after 60 seconds of each of Nonwovens 1 to 4 was 0 mm.

The contact angle of each of the four nonwovens was measured.

**Table 1:** Contact Angle of Nonwoven 1-4

|  | Contact Angle [°] |
|---|---|
| Nonwoven 1 | 93.0 |
| Nonwoven 2 | 86.8 |
| Nonwoven 3 | 37.5 |
| Nonwoven 4 | 28.6 |

The samples were formed to mimic the condition of folding over an extended portion the outer web over the inner web, such that the sample tested according to the test method below had three layers, namely an inner web sandwiched between two outer webs (i.e. the outer web and the extended portion of the outer web). The samples were formed without elastic strand in between, and the inner and outer web were attached to each other with 5 g/m$^2$ adhesive applied in spiral pattern. The samples are of squared shape with 4 cm dimension.

**Table 2:** Test Results for Residual Fluid

|  | Comparative Example 1 | Comparative Example 2 | Example 1 |
|---|---|---|---|
| outer web / extended, folded over portion of the outer web) | Nonwoven 2 | Nonwoven 4 | Nonwoven 4 |
| inner web | Nonwoven 1 | Nonwoven 1 | Nonwoven 3 |
| Residual Fluid on Petri dish [mg] *) | 215.73 (8.1) | 24.33 (1.4) | 20.40 (2.4) |
| Residual Fluid on Petri dish [%] | 89.9 | 10.3 | 8.6 |
| *) numbers in brackets indicate standard deviation | | | |

The test protocol above aims at mimicking in-use situation of the skin, covered by sweat and being in contact with the elastic laminate including an extended, folded over portion of the outer web: In this set up the Petri dish is mimicking the skin. The lower is the residual amount of fluid on Petri dish, both absolute and percentual, the better is the sweat management belt performance. The invention example 1, according to the test method above, shows an improvement compared to the 2 comparative examples.

**Claims**

1. A wearable article having a longitudinal direction extending from a front waist edge to a back waist edge, and a transverse direction extending perpendicular to the longitudinal direction,

   the wearable article comprising an elastic laminate which forms an elastic belt (40) with a front belt (84) and a back belt (86) which are discontinuous in the longitudinal direction of the article and wherein left and right transverse edges of the front belt (84) and the back belt (86) are joined by side seams to form two leg opening and such that the front and back waist edge jointly form a continuous waist opening,
   the elastic laminate comprising an inner web (94) and an outer web (92) being in face to face relationship with each other, the inner web (94) and outer web (92) being comprised by the complete surface area of the elastic laminate, wherein the inner web (94) and the outer web (92) are each formed of a hydrophilic nonwoven web comprising at least 90 weight-%synthetic fibers based on the total weight of the respective inner web (94) and outer web (92), wherein, at the front and back waist edge, the elastic laminate comprises a fold over region wherein the outer web (92) is extended beyond the elastic laminate, and the extended portion of the outer web (92) is folded over the inner web, such that a portion of the elastic belt (40) comprises the inner web (94) sandwiched between the outer web (92) and the extended portion of the outer web (92), wherein the extended folded over portion (95, 93) has a longitudinal extension parallel with the longitudinal direction of the wearable article, the longitudinal extension of the extended folded over portion (95, 93) being from 20% to 70% of the total length of the side seams.

2. The wearable article of claim 1, wherein the inner web (94) and the outer web (92) each have a contact angle of less than 70°, as measured according to the test method set out below.

3. The wearable article of claim 1 or 2, wherein the synthetic fibers of the first web are thermoplastic fibers.

4. The wearable article of any of the preceding claims, wherein the synthetic fibers of the first web are selected from the group consisting of polyethylene, polypropylene, polyester, polylactic acid, and mixtures and combinations thereof.

5. The wearable article of any of the preceding claims, wherein elastic laminate does not comprise any sheets other than the inner web, the outer web (92) and the extended portion of the outer web (92).

6. The wearable article of any of the preceding claims, wherein a plurality of elastic strands is provided between the inner and outer web (92), the elastic strands extend along the transverse direction and are spaced apart from each other in the longitudinal direction of the article.

7. The wearable article of any of the preceding claims, wherein the inner and outer web (92) are both non-elastic.

8. The wearable article of any of the preceding claims, wherein the outer web (92) has a plurality of openings, such as apertures, in the outer web (92) at an Opening Rate of from about 5% to about 50%, preferably from about 5% to about 30%, according to the test method set out herein.

9. The wearable article of claim 8, wherein the outer web (92) has an Effective Opening Area of from 0.1 mm$^2$ to 25 mm$^2$, preferably from 0.1 mm$^2$ to 10 mm$^2$, according to the test method herein.

10. The wearable article of any of the preceding claims, wherein the inner web (94) has a plurality of openings, such as apertures, at an Opening Rate of from 5% to 30%, preferably from 5 % to 15 %, and the first web has an Effective Opening Area of from 0.1 mm$^2$ to 25 mm$^2$, preferably from 0.1 mm$^2$ to 10 mm$^2$, according to the test method herein.

11. The wearable article of any of the preceding claims and comprising a central chassis (38); the center of the front belt (84) being joined to a front waist panel (52) of the central

chassis (38), the center of the back belt (86) being joined to a back waist panel (54) of the central chassis (38), and the remainder of the central chassis (38) between the front and back waist panel (54) forming a crotch region (30), the front and back belt (86) each having a left side panel and a right side panel (82) where the central chassis (38) does not overlap;
the central chassis (38) comprising an outer cover layer (42) on the garment-facing surface and a backsheet (60) attached to the inner surface of the outer cover layer; wherein the longitudinal length of the outer cover layer (42) is longer than the longitudinal length of the crotch region (30) and shorter than the longitudinal length of the backsheet (60).

**Patentansprüche**

1. Tragbarer Artikel, der eine Längsrichtung, die sich von einem vorderen Taillenrand zu einem hinteren Taillenrand erstreckt, und eine Querrichtung, die sich senkrecht zu der Längsrichtung erstreckt, aufweist,

der tragbare Artikel umfassend ein elastisches Laminat, das einen elastischen Gürtel (40) mit einem vorderen Gürtel (84) und einem hinteren Gürtel (86) ausbildet, die in Längsrichtung des Artikels unterbrochen sind, und wobei der linke und der rechte Querrand des vorderen Gürtels (84) und des hinteren Gürtels (86) durch Seitennähte verbunden sind, um zwei Beinöffnungen auszubilden, und derart, dass der vordere und der hintere Taillenrand gemeinsam eine zusammenhängende Taillenöffnung ausbilden,
das elastische Laminat umfassend eine innere Bahn (94) und eine äußere Bahn (92), die in einander zugewandter Beziehung sind, wobei die innere Bahn (94) und die äußere Bahn (92) in dem gesamten Oberflächenbereich des elastischen Laminats umfasst sind,
wobei die innere Bahn (94) und die äußere Bahn (92) jeweils aus einer hydrophilen Vliesbahn ausgebildet sind, umfassend mindestens zu 90 Gew.-% synthetische Fasern, bezogen auf das Gesamtgewicht der jeweiligen inneren Bahn (94) und äußeren Bahn (92),
wobei das elastische Laminat an dem vorderen und dem hinteren Taillenrand einen Umschlagbereich umfasst, wobei die äußere Bahn (92) über das elastische Laminat hinaus verlängert ist und der verlängerte Abschnitt der äußeren Bahn (92) über die innere Bahn gefaltet ist, derart, dass ein Abschnitt des elastischen Gürtels (40) die innere Bahn (94) umfasst, die zwischen der äußeren Bahn (92) und dem verlängerten Abschnitt der äußeren Bahn (92) in Sandwichform angeordnet ist, wobei der verlängerte Umschlagbereich (95, 93) eine Längsverlängerung parallel zu der Längsrichtung des tragbaren Artikels aufweist und die Längsverlängerung des verlängerten Umschlagbereichs (95, 93) von 20 % bis 70 % der Gesamtlänge der Seitennähte beträgt.

2. Tragbarer Artikel nach Anspruch 1, wobei die innere Bahn (94) und die äußere Bahn (92) jeweils einen Kontaktwinkel von weniger als 70 ° aufweisen, gemessen gemäß dem unten dargelegten Prüfverfahren.

3. Tragbarer Artikel nach Anspruch 1 oder 2, wobei die synthetischen Fasern der ersten Bahn Thermoplastfasern sind.

4. Tragbarer Artikel nach einem der vorstehenden Ansprüche, wobei die synthetischen Fasern der ersten Bahn aus der Gruppe ausgewählt sind, bestehend aus Polyethylen, Polypropylen, Polyester, Polymilchsäure und Mischungen und Kombinationen davon.

5. Tragbarer Artikel nach einem der vorstehenden Ansprüche, wobei das elastische Laminat keine beliebigen Lagen als die innere Bahn, die äußere Bahn (92) und den verlängerten Abschnitt der äußeren Bahn (92) umfasst.

6. Tragbarer Artikel nach einem der vorstehenden Ansprüche, wobei eine Vielzahl von elastischen Strängen zwischen der inneren und äußeren Bahn (92) bereitgestellt ist, die sich entlang der Querrichtung erstrecken und in Längsrichtung des Artikels voneinander beabstandet sind.

7. Tragbarer Artikel nach einem der vorstehenden Ansprüche, wobei sowohl die innere als auch die äußere Bahn (92) nicht elastisch sind.

8. Tragbarer Artikel nach einem der vorstehenden Ansprüche, wobei die äußere Bahn (92) eine Vielzahl von Öffnungen, wie Aperturen, in der äußeren Bahn (92) bei einer Öffnungsrate von etwa 5 % bis etwa 50 %, vorzugsweise von etwa 5 % bis etwa 30 %, gemäß dem hierin dargelegten Prüfverfahren aufweist.

9. Tragbarer Artikel nach Anspruch 8, wobei die äußere Bahn (92) eine wirksame Öffnungsfläche von 0,1 mm$^2$ bis 25 mm$^2$, vorzugsweise von 0,1 mm$^2$ bis 10 mm$^2$, gemäß dem Prüfverfahren hierin aufweist.

10. Tragbarer Artikel nach einem der vorstehenden Ansprüche, wobei die innere Bahn (94) eine Vielzahl von Öffnungen, wie Aperturen, bei einer Öffnungsrate von 5 % bis 30 %, vorzugsweise von 5 % bis 15 % aufweist, und die erste Bahn eine wirksame Öffnungsfläche von 0,1 mm$^2$ bis 25 mm$^2$, vorzugsweise von 0,1 mm$^2$ bis 10 mm$^2$, gemäß dem Prüfverfahren hierin aufweist.

11. Tragbarer Artikel nach einem der vorstehenden Ansprüche und umfassend eine mittige Grundeinheit (38); wobei die Mitte des vorderen Gürtels (84) mit einem vorderen Taillenfeld (52) der mittigen Grundeinheit (38) verbunden ist, die Mitte des hinteren Gürtels (86) mit einem hinteren Taillenfeld (54) der mittigen Grundeinheit (38) verbunden ist und der Rest der mittigen Grundeinheit (38) zwischen dem vorderen und hinteren Taillenfeld (54) einen Schrittbereich (30) ausbildet, wobei der vordere und der hintere Gürtel (86) jeweils ein linkes Seitenfeld und ein rechtes Seitenfeld (82) aufweisen, wo die mittige Grundeinheit (38) nicht überlappt;
die mittige Grundeinheit (38) umfassend eine Außenmantelschicht (42) auf der Oberfläche, die einem Kleidungsstück zugewandt ist, und eine Unterschicht (60), die an die Innenoberfläche der Außenmantelschicht gebunden ist; wobei die Längslänge der Außenmantelschicht (42) länger als die Längslänge des Schrittbereichs (30) und kürzer als die Längslänge der Unterschicht (60) ist.

**Revendications**

1. Article portable ayant une direction longitudinale s'étendant à partir d'un bord de taille avant jusqu'à un bord de taille arrière, et une direction transversale s'étendant perpendiculairement à la direction longitudinale,

   l'article portable comprenant un stratifié élastique qui forme une ceinture élastique (40) avec une ceinture avant (84) et une ceinture arrière (86) qui sont discontinues dans la direction longitudinale de l'article et dans lequel des bords transversaux gauche et droit de la ceinture avant (84) et de la ceinture arrière (86) sont joints par des coutures latérales pour former deux ouvertures de jambe et de telle sorte que les bords de taille avant et arrière forment conjointement une ouverture de taille continue,
   le stratifié élastique comprenant un voile interne (94) et un voile externe (92) étant en relation face à face, le voile interne (94) et le voile externe (92) étant compris par la surface totale du stratifié élastique,
   dans lequel le voile interne (94) et le voile externe (92) sont chacun formés d'un voile non tissé hydrophile comprenant au moins 90 % en poids de fibres synthétiques en fonction du poids total du voile interne (94) et du voile externe (92) respectifs,
   dans lequel, au niveau des bords de taille avant et arrière, le stratifié élastique comprend une région de rabattement dans lequel le voile externe (92) est étendue au-delà du stratifié élastique, et la partie étendue du voile externe (92) est rabattue sur le voile interne, de telle sorte qu'une partie de la ceinture élastique (40) comprend le voile interne (94) pris en sandwich entre le voile externe (92) et la partie étendue du voile externe (92), dans lequel la partie étendue rabattue (95, 93) a une extension longitudinale parallèle à la direction longitudinale de l'article portable, l'extension longitudinale de la partie étendue rabattue (95, 93) allant de 20 % à 70 % de la longueur totale des coutures latérales.

2. Article portable selon la revendication 1, dans lequel le voile interne (94) et le voile externe (92) ont chacun un angle de contact inférieur à 70°, tel que mesuré selon le procédé de test présenté ci-dessous.

3. Article portable selon la revendication 1 ou 2, dans lequel les fibres synthétiques du premier voile sont des fibres thermoplastiques.

4. Article portable selon l'une quelconque des revendications précédentes, dans lequel les fibres synthétiques du premier voile sont choisies dans le groupe constitué de polyéthylène, polypropylène, polyester, acide polylactique, et de mélanges et de combinaisons de ceux-ci.

5. Article portable selon l'une quelconque des revendications précédentes, dans lequel le stratifié élastique ne comprend aucune feuille autre que le voile interne, le voile interne (92) et la partie étendue de la voile externe (92).

6. Article portable selon l'une quelconque des revendications précédentes, dans lequel une pluralité de fils élastiques sont prévus entre les voiles interne et externe (92), les fils élastiques s'étendant le long de la direction transversale et étant espacés les uns des autres dans la direction longitudinale de l'article.

7. Article portable selon l'une quelconque des revendications précédentes, dans lequel les voiles interne et externe (92) sont tous deux non élastiques.

8. Article portable selon l'une quelconque des revendications précédentes, dans lequel le voile externe (92) a une pluralité d'ouvertures, telles que des perforations, dans le voile externe (92) à un taux d'ouverture d'environ 5 % à environ 50 %, de préférence d'environ 5 % à environ 30 %, selon le procédé de test présenté ici.

9. Article portable selon la revendication 8, dans lequel le voile externe (92) a une zone d'ouverture effective de 0,1 mm$^2$ à 25 mm$^2$, de préférence de 0,1 mm$^2$ à 10 mm$^2$, selon le procédé de test présenté ici.

10. Article portable selon l'une quelconque des revendications précédentes, dans lequel le voile interne (94) a une pluralité d'ouvertures, telles que des perforations, à un taux d'ouverture de 5 % à 30 %, de préférence de 5 % à 15 %, et le premier voile a une zone d'ouverture effective de 0,1 mm$^2$ à 25 mm$^2$, de préférence de 0,1 mm$^2$ à 10 mm$^2$, selon le procédé de test présenté ici.

11. Article portable selon l'une quelconque des revendications précédentes et comprenant un châssis central (38) ; le centre de la ceinture avant (84) étant joint à un empiècement de taille avant (52) du châssis central (38), le centre de la ceinture arrière (86) étant joint à un empiècement de taille arrière (54) du châssis central (38), et le reste du châssis central (38) entre l'empiècement de taille avant et arrière (54) formant une région d'entrejambe (30), la ceinture avant et arrière (86) ayant chacune un empiècement latéral gauche et un empiècement latéral droit (82) où le châssis central (38) ne se chevauche pas ;

le châssis central (38) comprenant une couche de recouvrement externe (42) sur la surface tournée vers le vêtement et une feuille de fond (60) liée à la surface interne de la couche de recouvrement externe ; dans lequel la longueur longitudinale de la couche de recouvrement externe (42) est plus longue que la longueur longitudinale de la région d'entrejambe (30) et plus courte que la longueur longitudinale de la feuille de fond (60).

FIG. 1A

**FIG. 1B**

FIG. 2A  FIG. 2B  FIG. 2C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2017012319 A
- JP 2017113186 A
- WO 2019169989 A
- US 20190274895 A
- CN 102316838
- WO 2011087503 A
- US 5628097 A
- WO 201673712 A
- WO 200617718 A

### Non-patent literature cited in the description

- Contact Angle, Wettability and Adhesion. 1964